# EUROPEAN PATENT SPECIFICATION

(11) **EP 2 475 321 B1**
(45) Date of publication and mention of the grant of the patent: **11.03.2015**
(21) Application number: 09788159.3
(22) Date of filing: 11.09.2009
(51) Int. Cl.: A61B 18/24, A61B 17/11

(54) **A LASER CATHETER SYSTEM FOR BYPASS SURGERY**
LASERKATHETERSYSTEM FÜR DIE BYPASS-CHIRURGIE
SYSTÈME CATHÉTER À LASER POUR OPÉRATIONS DE PONTAGE

(43) Date of publication of application: 18.07.2012
(73) Proprietor: Corvasco Medical B.V., 3584 CM Utrecht (NL)
(72) Inventor: TULLEKEN, Cornelis, Antonius, Franciscus, NL-3511 XP Utrecht (NL); VAN DOORMAAL, Theodorus, Petrus, Cornelis, NL-3732 DK DE Bilt (NL)
(74) Representative: EP&C
(86) International application number: PCT/NL2009/000178
(87) International publication number: WO 2011/031131

(56) References cited:
- EP-A- 0 311 295
- EP-A- 1 967 152
- WO-A-00/19920
- WO-A-01/15618
- WO-A-03/037203
- WO-A-03/057060
- US-A- 5 989 243
- US-A1- 2009 125 007

## Description

### Field of the invention

The present invention relates to a catheter system and more particularly to a catheter system comprising a laser catheter provided with a fibre bundle of optical fibres having distal ends defining a light emitting surface for emitting a light beam in the distal direction of the catheter and a laser apparatus, comprising one or more lasers for supplying light to the optical fibres.

### Background of the invention

A laser catheter for bypass surgery is known from EP 750,476. This document describes the use in the ELANA ® (Excimer Laser Assisted Non-occlusive Anastomosis) operating technique. For this technique, one requires a catheter and a ring, which are jointly called Elana ® Arteriotomy System.

The catheter disclosed in EP 750,476 is used for performing an ETS-anastomosis (ETS = End To Side) between a graft vessel and a target vessel. The graft is fixed with an end to the side of the target vessel, while the blood flow through the target vessel, also called recipient vessel, is not interrupted, i.e. blood continues to flow through the target vessel while performing the anastomosis. For this purpose, first the graft vessel is fixed to the target vessel and subsequently, after this fixation is established, the flow connection between the target vessel and graft vessel is made by removing the part of the wall of the target vessel which lies in front of the fixed end of the graft vessel. Said part of the wall of the target vessel is removed by means of a tubular arrangement of optical fibres emitting a tubular bundle of laser light beam originating from the fibres and a suction gripper provided inside the tubular arrangement of optical fibres. The tubular bundle of the laser light beam ablates a circle into the wall of the target vessel, resulting in a circular passage connecting the lumens of the graft vessel and target vessel. The circular wall part of the target vessel - i.e. the part lying inside said circle - is gripped by the suction gripper and removed together with the withdrawal of the catheter after the ablation operation. The distal ends of the optical fibres of this known laser catheter define a circle extending in a plane essentially perpendicular to the longitudinal axis of the catheter. During use the laser catheter extends perpendicular to the target vessel, resulting in a perpendicular ETS-anastomosis with a circular passage between the graft vessel and target vessel.

When applying this technique, it is important that the circular wall part of the target vessel is removed completely during operation. If not, additional actions have to be taken to prevent the circular wall part from entering the target vessel or from obstructing the blood flow.

A catheter system for vascular laser treatments is known from WO-03/057060. This document discloses a device for treatment of vascular occlusions, comprising an excimer laser pulse generator and a laser catheter having optical fibres for guiding the laser pulses to the distal end of the catheter. The laser catheter, containing a central guide wire lumen, is introduced through a guide catheter. The distal tip of the laser catheter is placed in contact with the thrombus to be removed. The laser is activated to perform thrombus removal by sending ultraviolet laser through the catheter against the thrombus and the thrombus is removed by ablation.

### Summary of the invention

The invention is defined in the appended set of claims.

The object of the present invention is to provide a laser catheter for bypass surgery, such as especially for the ELANA technique, that will increase the effectiveness of the operation, which is defined as the percentage of complete cut of the so called 'flap'. The flap is the wall part (of the target vessel) which is to be cut away from the wall of the target vessel for providing the connection from the target vessel to the graft vessel.

According to the invention, this object is achieved by providing a catheter system according to claim 1, comprising a laser catheter provided with a fibre bundle of optical fibres having distal ends defining a light emitting surface for emitting a light beam in the distal direction of the catheter, and a laser apparatus, comprising one or more lasers for supplying light to the optical fibres, wherein the system is preset or adjusted for emitting a pulsating light beam with an ablation power of at least 40 mJ/mm² per pulse at the location of the light emitting surface. The laser apparatus is an excimer laser which provides ultraviolet light having a wavelength in the range of 240-340, or about 308 nm. The pulsating light beam has a pulsating frequency in the range of 25-80 Hz. The pulsating light beam has a pulse width in the range of 50-300 ns at Full Width at Half Maximum (F.W.H.M). The catheter system according to the invention further comprises a timing device for setting emitting intervals and break intervals at predefined time values, wherein the catheter system is arranged to emit the pulsating light beam during at least two emitting intervals, wherein each two consecutive intervals are separated by a break interval of at least 1 s, during which emitting the pulsating light beam is interrupted. In former techniques, the power is normally limited to 20 mJ/mm² or less to prevent the light beam from cutting through the whole target vessel, i.e. through both sides of the vessel. Emitting the light bundle of pulsating light beam from the light emitting surface with an ablation power of at least 40 mJ/mm² per pulse or more preferably at least 45 mJ/mm² per pulse, results in a more reliable and better cutting away of tissue, which means that the flap is cut away more effectively.

The area used for calculating the ablation power is the area of the light emitting surface, this being the sum of all the distal end areas of all optical fibres. The ablation power is measured at the location of the light emitting surface, i.e. the energy of the light beam actually emitted by the distal ends of the optical fibres is measured.

Surprisingly, despite the higher ablation power, cutting through both sides of the vessel does not occur, only the vessel wall directly adjacent the target vessel and light emitting surface is cut. It is assumed that the blood flowing through the vessel adequately prevents the light beam form cutting through the vessel at the opposite site. The higher ablation power of at least 40 mJ/mm² per pulse further guarantees that the flap is fully cut away without leaving any remnants connected to the target vessel.

An advantage of providing a pulsating light beam is that with a pulsating light beam high energies in a short time can dissipate, resulting in cutting tissue, without heating up a larger part of the tissue. Such heating could cause burning damages.

The dissipation of energy at the surface of the target vessel may create gasses. These gasses may cause the surface of the target vessel wall to move slightly. An advantage of emitting the pulsating light beam during at least two emitting intervals, separated by a break interval, is that during the break interval the surface of the target vessel can stretch back to its preferred position. It is known that cutting is performed most effectively when the surface of the target vessel is positioned adjacent to and stretched along the light emitting surface. Allowing the surface of the target to stretch back will increase the effectiveness of the cutting in the consecutive emitting interval. Also gasses created by the ablation can disperse or disappear during the break interval and an abundance or built up of gasses is prevented.

According to a second aspect, the object of the invention is achieved by providing a catheter system comprising a laser catheter provided with a fibre bundle of optical fibres having distal ends defining a light emitting surface for emitting a pulsating light beam in the distal direction of the catheter, a laser apparatus, comprising one or more lasers for supplying light to the optical fibres, and an adjuster for setting an ablation power of the pulsating light beam, a power sensor for measuring the ablation power and for generating a power signal representative for the measured ablation power and a controller for controlling the adjuster in response to the power signal to set said ablation power at least 40 mJ/mm² per pulse at the location of the light emitting surface.

The ablation power at the location of the light emitting surface is among other factors dependent on the attenuation characteristics of the optical fibres used. The fibre bundles of optical fibres are typically used only once or a few times, while the laser apparatus is used many times and thus with several different fibre bundles. It may therefore be advantageous to adjust the ablation power for each combination of a laser apparatus and a fibre bundle of optical fibres.

In a further embodiment of the invention, the ablation power is in the range of 40-100 mJ/mm² per pulse, preferably in the range of 40-60 mJ/mm² per pulse. An advantage of an ablation power in this range is that the so called 'flap' is cut away very effectively. Increasing the ablation power beyond this range on one hand may have disadvantageous effects, for example on the blood flowing through the target vessel, while on the other hand the effectiveness of cutting away the flap does not appear to improve further.

In another embodiment, the optical fibres of the fibre bundle are arranged in a tubular configuration to define said light emitting surface as being ring shaped. Since an ETS-anastomosis (ETS = End To Side) is usually done with a tubular shaped graft, it may be advantageous to provide a fibre bundle arranged in a tubular configuration for easy insertion. The ring shaped light emitting surface will result in the flap having a circular shape, and therefore the connecting opening between the graft wall and the target wall will also be circular.

In a further embodiment the timing device further comprises an input device for inputting predefined time values for the emitting intervals and break intervals. In preferred embodiments, the emitting intervals are at least 2 s, or preferable in the range of 2-5 s. The break intervals are preferably in the range of 1-5 s.

In another embodiment the pulsating light beam has a pulsating frequency of around 40 Hz.

In order to remove the flap of the target vessel after ablation of the ring of tissue, it is advantageous to provide the distal part of the catheter with a gripper for gripping tissue inside the tubular bundle of the light beam. According to the invention, the gripper preferably comprises a hollow channel extending within the fibre bundle of optical fibres and connectable to a vacuum source, wherein the distal end of the channel defines a suction mouth. In order to ensure a firm gripping of the cut out part of the wall tissue, it is according to the invention advantageous to arrange the suction mouth at a distance proximally from the light emitting surface and so that it defines a suction surface parallel to the light emitting surface. The suction surface parallel to the light emitting surface ensures an easy gripping of the cut out part, i.e. the flap, all over its surface.

The stop surface forms locally a radial bulge on the outer surface of the catheter. When the catheter is inserted into a graft vessel, this bulge will be visible as a bulge in the wall of the graft vessel or at least tangible with the fingers of the surgeon. This enables the surgeon to control or correct the orientation and the position of the light emitting surface.

In another embodiment of the invention, the catheter system further comprises a ring member having dimensions adapted for, on the one hand, insertion of the distal end of the fibre bundle of optical fibres through said ring member and for, on the other hand, preventing passage of the stop surface through said ring member.

In a further embodiment, the catheter system comprises a graft vessel having diameter dimensions allowing, on the one hand, passage of the laser catheter and, on the other hand, insertion through said ring member. This graft vessel can be a donor vessel obtained from the patient or another person, but it can also be an artificial graft vessel manufactured from biological and/or non-biological material.

Before connecting the graft vessel to the target vessel, the graft vessel will be prepared for the bypass procedure by inserting one end of the graft vessel through the ring member and folding back the end of the graft vessel around the ring member. Before using the laser catheter, this folded end of the graft vessel with the ring member located in the inside of the fold, will be attached to the wall of the target vessel. Subsequently, when the laser catheter has been introduced into the graft vessel and the laser operation is performed, the ring member will prevent the laser catheter from advancing too far into the target vessel as soon as the stop surface comes to rest onto the ring member.

In a further embodiment in which the one end of the graft vessel is inserted through said ring member and folded back over said ring member, the angle between the graft vessel and the part of the graft vessel folded back is in the range of 90 - 180 degrees. It can be advantageous to fold a part of the graft vessel. In that way it can easily be connected to the target vessel.

### Brief description of the drawings

Further advantageous embodiments of the assembly according to the invention are described in the claims and in the following description with reference to the drawing, in which:
Figure 1 shows a laser catheter of a catheter system according to the invention, wherein figure 1A is a longitudinal view in cross section and figure 1B shows an end view according to arrow IB in figure 1A.
Figure 2 shows a part of a catheter system according to the invention and a sequence of steps in a ETS-anastomosis procedure according to the invention, wherein figure 2 is sub-divided into the figures 2a, 2b, 2c and 2d, which each show a different step.
Figure 3 shows a schematic overview of the devices involved with manipulating the laser light.
Figure 4a/b show a power output diagram of the pulsating light beam.

### Detailed description of the invention

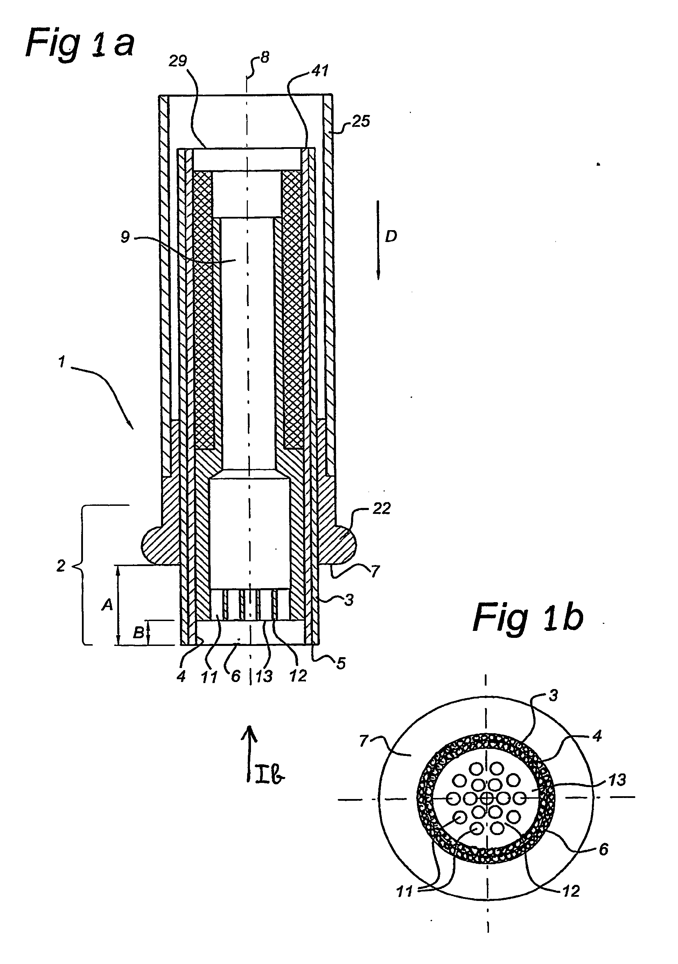

Figure 1 shows a laser catheter 1. The distal part 2 of the laser catheter 1 is provided with a fibre bundle arrangement 3 of optical fibres 4. The optical fibres 4 have distal ends 5, which together define a light emitting surface 6. When a pulsating laser source is connected to the proximal ends 41 of the optical fibres 4, a pulsating light beam will emit from each of these distal ends 5 of the optical fibres 4. The distal ends of the optical fibres 4 extend parallel to the longitudinal axis 8 of the catheter, so that the emitted light beam will extend parallel to the longitudinal axis 8 in the distal direction indicated by arrow D.

When the fibre bundle comprises a tubular bundle of optical fibres, the light emitting surface will be ring-shaped and the pulsating light beam bundle will result in a tubular light beam in the distal direction D of the catheter. However, other arrangement of optical fibres are also possible, for example with optical fibres randomly distributed in ring-shape or a massive fibre bundle of fibres. The latter would enable the ablation of a complete disc - or another shape - without leaving a core. In the following an embodiment is described with a tubular bundle of optical fibres.

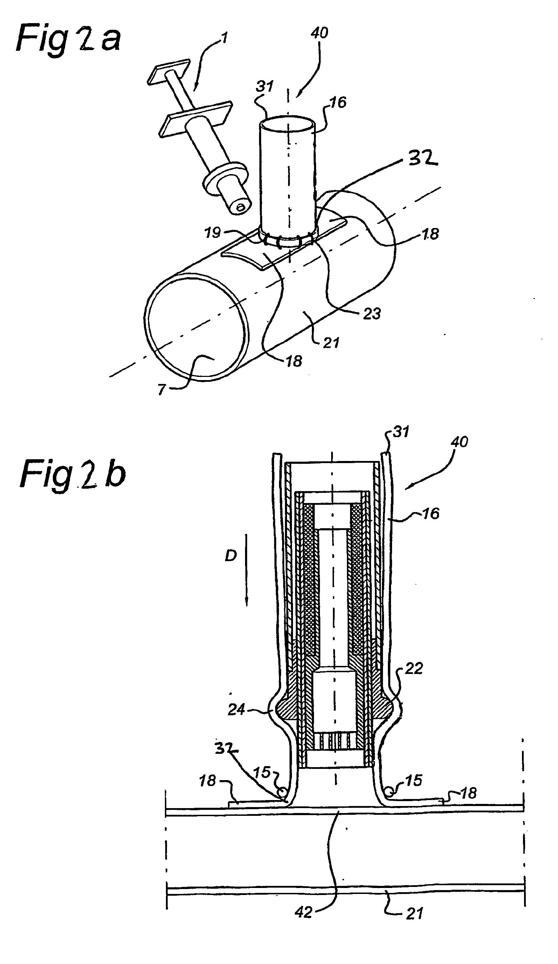

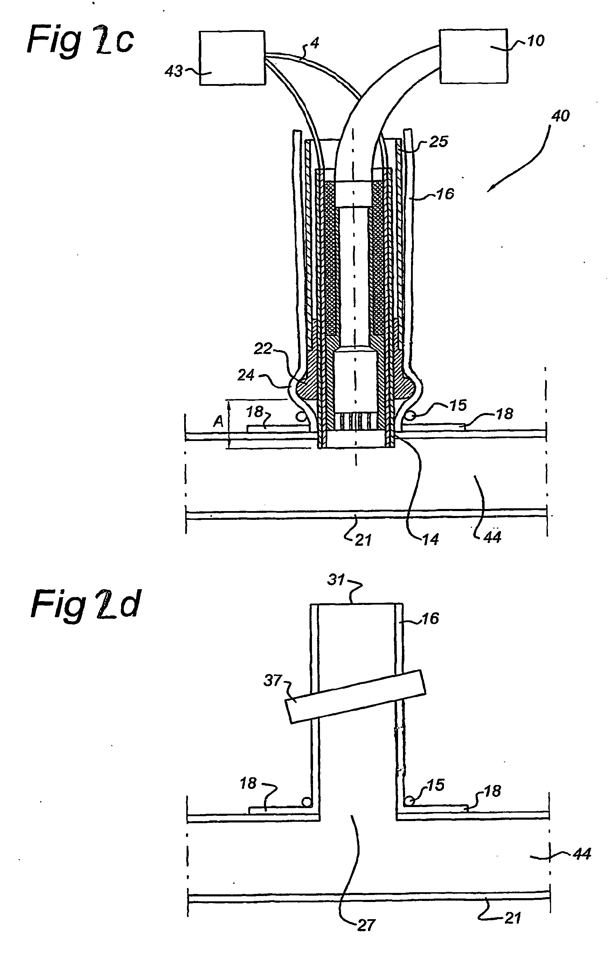

The laser catheter 1 further comprises a casing 25 surrounding the tubular fibre bundle 3 of optical fibres. The tubular fibre bundle 3 of optical fibres 4 encloses a channel 9. The proximal end 29 of the channel 9 can be connected to a vacuum source 10 (see figure 2c) in order to apply a suction force to the channel 9. The distal end of the channel 9 is provided with a plate, defining the suction surface 13 and provided with suction apertures 11. The distal end of the channel 9 thus forms a suction mouth 12, which acts as a gripper when vacuum is applied at the proximal end 29 of channel 9. The suction mouth 11 is provided at a distance B proximally from the light emitting surface 6. This distance B will at least be about the thickness of the wall of the target vessel (21, in figure 2).

However, in some cases, for example if a complete disc of tissue is to be cut away, there is no need for the use of a gripper.

The distal end of the casing 25 is provided with a radial bulge 22. The distal side of the radial bulge 22 forms a stop surface 7. This stop surface lies proximally at a distance A from the light emitting surface 6. This distance A will at least be about twice the wall thickness of the first vessel 16 (which follows from figure 2c discussed below) plus the wall thickness of the target vessel 21 (which also follows from figure 2c).

In order to ensure a good gripping of the flap 14 (figure 2c) - the term 'flap' indicates 'the tissue part separated after ablation of a piece of tissue' - by the suction mouth 12, the suction surface 13 of the suction mouth extends parallel to the light emitting surface 6.

The bulge 22 with the stop surface 7 also extends parallel to the light emitting surface 6. It will be clear that the bulge 22 is preferably a bulge extending continuously around the catheter, but that it may also be a discontinuous bulge. The outer diameter of the bulge 22 is larger than the inner diameter of the ring member 15. This reliably prevents the distal part of the catheter from being inserted too far into the target vessel.

The distal ends 5 of the optical fibres 4 lie closely packed together with the longitudinal walls of adjacent fibres against each other to form together a tubular arrangement 3 having a circular cross-section as can be seen in figure 1B. However, with different arrangement of optical fibres, differently shaped cross-sections are also possible. The distal end faces of all the optical fibres 4 together define an essentially flat light emitting surface 6, which extends perpendicular to the longitudinal axis 8 of the catheter.

Due to the distal ends 5 of the optical fibres being closely packed, the light beam, which is emitted when a laser light source is connected, forms an essentially continuous bundle, for example a circular bundle, which is capable of ablating a continuous piece of tissue from a target vessel.

Referring to figures 2a-2d, an ETS-anastomosis procedure with the above catheter will be described.

Figure 2a shows a first step. The distal end 32 of the graft vessel 16 is attached to the side wall of the target vessel 21, leaving the part 42 (see figure 2b) of the wall tissue of the target vessel 21 in front of the lumen of the graft vessel 16 intact so that the blood flow in the target vessel 21 can be left undisturbed. An advantage may be that attaching the graft to the target vessel does not require occlusion of the target vessel. The graft vessel 16 can be fixed to the target vessel 21 by any suitable connection technique, such as connection techniques known from the prior art which preferably do not require the part 42 of wall tissue to be removed before. Figure 2a shows for example a suture 23 enclosing the ring member 15 as well as piercing through the graft vessel 16 and the target vessel 21. Instead of a suture 23 also a staple could be used. Further, as is shown in figure 2, the flanges 18 can be used for establishing a good connection to the target vessel 21. The flanges 18 can for example be glued to the target vessel 21.

After a firm and sufficiently leak tight connection between the graft vessel 16 and target vessel 21 has been established, the laser catheter 1 of figure 1 is inserted into the proximal end 31 of the graft vessel 16, see figure 2b. As can be seen in figure 2b, the bulge 22 on the outer circumference of the laser catheter 1 causes a similar bulge 24 in the wall of the graft vessel 16. This bulge 24 allows the surgeon to see how far the catheter is advanced in the graft vessel 16.

The laser catheter 1 is advanced distally (arrow D in figure 2b) until the light emitting surface 6 contacts the wall part 42 to be removed from the target vessel. In case not already done before, the channel 9 and optical fibres 4 are, subsequently, connected to a vacuum source 10 and laser light source , respectively. A vacuum is applied to the channel 9 and the laser procedure is started. Laser light is supplied to the optical fibres 4. Thus doing, the light emitting surface 6 gradually advances forward through the wall of the target vessel until said surface 6 faces or protrudes into the lumen 44 of the target vessel 21. The so called flap 14 is gripped by the suction mouth 11. At this moment, the laser procedure is finished and the laser light source can be switched off. Subsequently, the laser catheter is retracted in the direction opposite to arrow D, whilst the flap 14 is being removed by the suction gripper 11.

As soon as the laser catheter has been retracted over a sufficient distance, a clip 37 (figure 4d) or other closure is placed on the graft vessel 16 in order to close it off. Blood will be allowed to enter the graft vessel through the aperture 27, but will not be able to pass the clip 37. The proximal end 31 of the graft vessel can be connected by a ETE-anastomosis (ETE = End To End) to another vessel, such as another graft vessel, or it can be connected by an ETS-anastomosis to the same or another target vessel.

The laser catheter and its use as described up to here in the 'detailed description' corresponds essentially to the laser catheter disclosed in EP 750,476. Next, more specifically the present invention will be addressed.

Referring to Figures 1 a and 1 b, the optical fibres 4 have distal ends 5, which together define a light emitting surface 6. In Figure 1b, the stop surface 7 of the bulge 22 can be seen around two rings of distal fibre ends 5 which together form the light emitting surface 6. According to the invention, the ablation power of the pulsating light beam emitted from the light emitting surface 6 is at least 40 mJ/mm² per pulse, or more preferably at least 45 mJ/mm² per pulse at the location of the light emitting surface. The power is measured at the location of the light emitting surface 6, where the light beam leaves the fibres. The area used for calculating the ablation power is the area of the light emitting surface, this being the sum of all the distal end areas of all optical fibres.

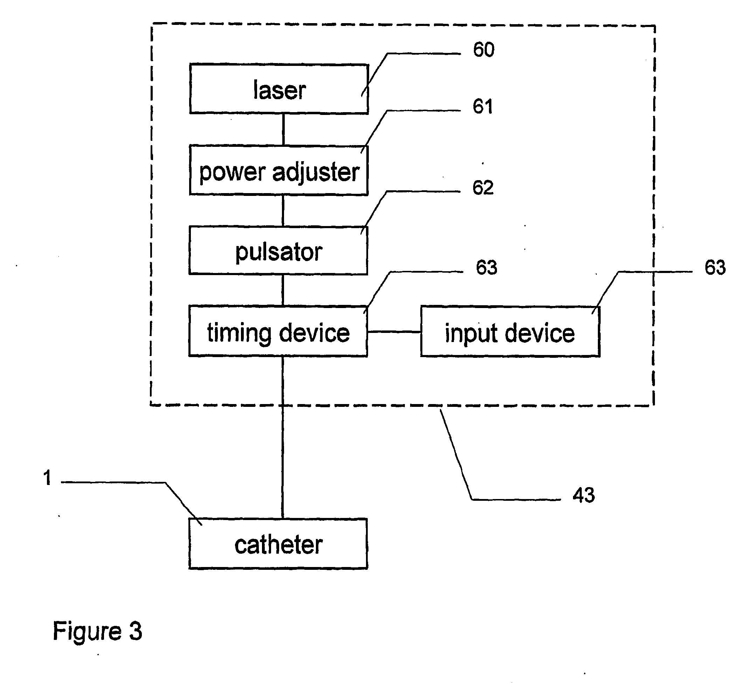

The power of the light beam can be adjusted in several ways. The laser apparatus itself can be provided with a power control for controlling the power of the lasers used. It is also possible that the transfer efficiency of the laser light through the fibres can be controlled, for example by changing the position or the angle between the laser emitting surface and the fibre receiving surface at the proximal side 41 of the fibres. In this way, the optical power of the laser is not completely transferred to the fibres. Yet another possibility would be to implement an optical dimmer in the optical fibres. In figure 3, a schematic view of the laser apparatus connected to the laser catheter 1 is presented, with a laser 60 and a power adjuster 61 depicted. The other functional parts of the assembly are discussed below.

In an embodiment, the catheter system comprises a laser catheter 1, a laser apparatus 60, an adjuster 61 for setting the ablation power of the pulsating light beam, a power sensor 65 for measuring the ablation power of the light beam and a controller 66 for controlling the adjuster 61 in response to the measurement of the power sensor 65.

When the catheter system is prepared for use, the laser apparatus 60 will first supply light with a laser power to the optical fibres. Because of the attenuation of the optical fibres and the adjuster 61, the ablation power at the location of the light emitting surface will be less than the laser power. To adjust the ablation power to its desired value, the catheter system is arranged for the following. First power sensor 65 measures the ablation power at the location of the light emitting surface. The controller 66 is arranged to compare this measured ablation power with a predefined power value requirement. A user of the catheter system, for example a surgeon, may act as the controller, when he or she adjusts the power of the laser apparatus manually after measuring the ablation power and reading the measured ablation power for example on a display. In case the measured ablation power is different from the predefined power value requirement, the controller, such as the user, operates the adjuster in such a manner that the attenuation of the adjuster is changed and that ablation power is increased when the measured ablation power is lower than the predefined power value requirement and that the ablation power is decreased when the measured ablation power is higher than the predefined power. Of course, the controller can also be an automatic device, for example implemented on a microprocessor.

In an embodiment according to the invention, the catheter system repeats the steps of measuring, comparing and adjusting until the measured ablation power meets the predefined power value requirement.

According to the invention, a high effectiveness of the surgery can be achieved when the predefined power value requirement is at least 40 mJ/mm² per pulse, preferably in the range of 40-100 mJ/mm² per pulse, or more preferably in the range of 40-60 mJ/mm² per pulse.

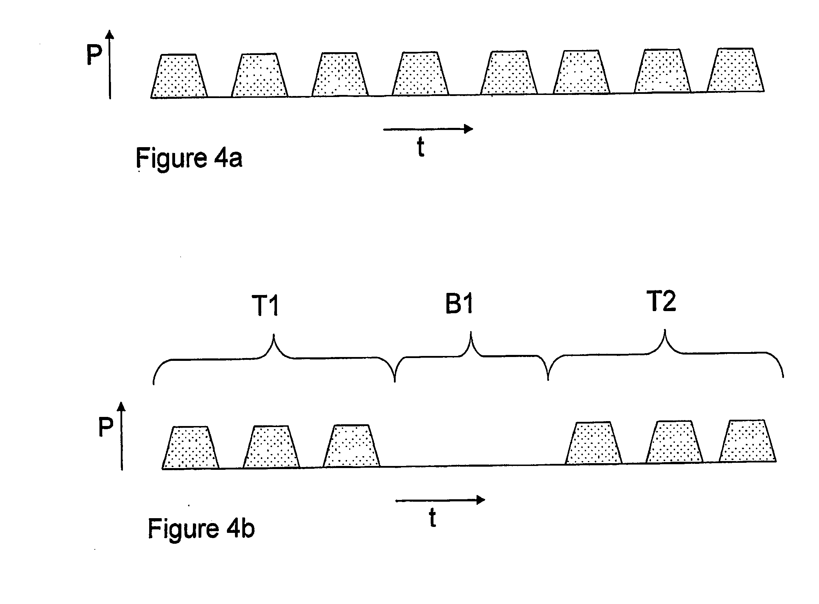

In figure 4a, a diagram shows, very schematically, the ablation power emitted at the location of the light emitting surface, in the direction indicated by E as function of the time in the direction indicated by t. The power is emitted during very short pulsating intervals. The pulsating frequency is e.g. at least 25 Hz, preferably in the range of 25-80 Hz.

The pulse width of the pulsating light beam is measured at full width at half maximum (F.W.H.M.) and is preferably in the range of 50-300 ns at F.W.H.M.

According to the invention, the catheter system is provided with a timing device 61 and is arranged to emit the tubular bundle of light beam during at least two emitting intervals, separated with a break interval. The power of the light emitted as a function of time is depicted very schematically in figure 4b. During the first interval T1, pulsating light is emitted. Only a few pulses are shown in figure 4b, in practice the number of pulses will be much larger in a interval. During the break interval B1, no light is emitted. And during the second interval T2, pulsating light is emitted again. Again, only a few pulses are shown in figure 4b, in practice the number of pulses will be much larger in a interval.

In a further embodiment the timing device 63 is further connected with an input device 64 for inputting time values for the emitting intervals and break intervals. This can be done for example by a numeric keyboard.

## Claims

1. A catheter system, comprising:
• a laser catheter (1) provided with a fibre bundle (3) of optical fibres (4) having distal ends (5) defining a light emitting surface (6) for emitting a pulsating light beam in the distal direction of the catheter (1); and
• a laser apparatus (43), comprising one or more lasers for supplying light to the optical fibres (4), wherein the laser apparatus (43) is an excimer laser providing ultraviolet light in the range of 240-340 nm;
wherein the pulsating light beam has a pulsating frequency in the range of 25-80 Hz; wherein the pulsating light beam has a pulse width in the range of 50-300 ns at F.W.H.M; wherein the system is preset or adjusted for emitting a pulsating light beam with an ablation power of at least 40 mJ/mm² per pulse at the location of the light emitting surface (6); wherein the catheter (1) system further comprises a timing device (63) for setting emitting intervals T1, T2) and break intervals (B1) at predefined time values; and
wherein the catheter (1) system is arranged to emit the pulsating light beam during said at least two emitting intervals (T1, T2), wherein each two consecutive emitting intervals (T1, T2) are separated by a break interval (B1) of at least 1 s, during which emitting the pulsating light beam is interrupted.

2. The catheter system according to claim 1, further comprising:
• an adjuster (61) for setting an ablation power of the pulsating light beam;
• a power sensor (65) for measuring the ablation power and for generating a power signal representative for the measured ablation power; and
• a controller for controlling the adjuster in response to the power signal to set said ablation power at a value of at least 40 mJ/mm² per pulse at the location of the light emitting surface (6).

3. The catheter system according to one of the preceding claims,
wherein the ablation power is in the range of 40-100 mJ/mm² per pulse, preferably in the range of 40-60 mJ/mm² per pulse.

4. The catheter system according to one of the preceding claims,
wherein
the optical fibres (4) of the fibre bundle (3) are arranged in a tubular configuration to define said light emitting surface (6) as being ring shaped.

5. The catheter system according to one of the preceding claims, wherein the timing device (63) further comprises an input device (63) for inputting predefined time values for the emitting intervals (T1, T2) and break intervals (B1).

6. The catheter system according to one of the preceding claim, wherein the emitting intervals (T1, T2) are each at least 2 s, preferably in the range of 2 - 5 s.

7. The catheter system according to one of the preceding claim, wherein the break interval (B1) is in the range of 1-5 s,

8. The catheter system according to one of the preceding claims, wherein the pulsating light beam has a pulsating frequency of around 40 Hz.

9. The catheter system according to one of the preceding claims,
wherein the distal part of the catheter (1) is provided with a gripper (11) for gripping tissue; wherein the gripper (11) comprises a hollow channel (9) extending within the fibre bundle (3) of optical fibres (4) and connectable to a vacuum source (10), the distal end of the channel defining a suction mouth (11); and
wherein the suction mouth (11) is arranged at a distance proximally from the light emitting surface (6) and defines a suction surface parallel to the light emitting surface (6).

10. The catheter system according to one of the preceding claims, wherein the laser catheter (1) comprises a stop surface (7) extending around the fibre bundle (3) of optical fibres (4) and facing in the distal direction, the stop surface (7) being arranged at a distance proximally from the light emitting surface (6).

11. The catheter system according to claim 10, further comprising a ring member having (15) dimensions adapted for, on the one hand, insertion of the distal end of the fibre bundle (3) of optical fibres (4) through said ring member (15) and for, on the other hand, preventing passage of the stop surface (7) through said ring member (15).

12. The catheter system according to claim 11, further comprising a graft vessel (16) having diameter dimensions allowing, on the one hand, passage of the laser catheter (1) and, on the other hand, insertion through said ring member (15).

13. The catheter system according to claim 12, wherein the graft vessel (16) is an artificial graft vessel.

14. The catheter system according to claim 12 or 13,
wherein one end of the graft vessel (16) is inserted through said ring member (15) and folded back over said ring member (15), and wherein the angle between the graft vessel (16) and the part (18) of the graft vessel folded back is in the range of 90 - 180 degrees.

15. The catheter system according to one of the preceding claims, wherein the light emitting surface (6) is essentially perpendicular to the longitudinal direction of the catheter (1).

## Patentansprüche

1. Kathetersystem, mit:
• einem Laserkatheter (1), der mit einem Faserbündel (3) aus optischen Fasern (4) versehen ist, welche distale Enden (5) haben, die eine Licht emittierende Oberfläche (6) zum Emittieren eines pulsierenden Lichtstrahls in die distale Richtung des Katheters (1) bilden; und
• einer Laservorrichtung (43) umfassend ein oder mehrere Laser zum Zuführen von Licht an die optischen Fasern (4), wobei die Laservorrichtung (43) ein Excimer-Laser ist, der ultraviolettes Licht im Bereich von 240 bis 340 nm liefert;
wobei der pulsierende Lichtstrahl eine Pulsfrequenz im Bereich von 25 bis 80 Hz hat;
wobei der pulsierende Lichtstrahl eine Pulsbreite im Bereich von 50 bis 300 ns bei FWHM hat;
wobei das System zum Emittieren eines pulsierenden Lichtstrahls mit einer Ablationsleistung von wenigstens 40 mJ/mm² pro Puls am Ort der Licht emittierenden Oberfläche (6) voreingestellt oder eingestellt ist;
wobei das Katheter (1)-System ferner eine Zeitsteuereinrichtung (63) zum Einstellen von Emissionsintervallen (T1, T2) und Unterbrechungsintervallen (B1) an vorbestimmten Zeitwerten umfasst; und
wobei das Katheter (1)-System so angeordnet ist, dass dieses den pulsierenden Lichtstrahl während der wenigstens zwei Emissionsintervalle (T1, T2) emittiert,
wobei alle zwei aufeinanderfolgenden Emissionsintervalle (T1, T2) durch ein Unterbrechungsintervall (B1) von 1 s getrennt sind, währenddessen ein Emittieren des pulsierenden Lichtstrahls unterbrochen ist.

2. Kathetersystem nach Anspruch 1, ferner mit:
• einer Einstelleinrichtung (61) zum Einstellen einer Ablationsleistung des pulsierenden Lichtstrahls;
• einem Leistungssensor (65) zum Messen der Ablationsleistung und zum Erzeugen eines Leistungssignals, das für die gemessene Ablationsleistung repräsentativ ist; und
• einer Steuereinrichtung zum Steuern der Einstelleinrichtung in Abhängigkeit von dem Leistungssignal, um die Ablationsleistung auf einen Wert von wenigstens 40 mJ/mm² pro Puls am Ort der Licht emittierenden Oberfläche (6) einzustellen.

3. Kathetersystem nach einem der vorhergehenden Ansprüche, in welchem die Ablationsleistung im Bereich von 40 bis 100 mJ/mm² pro Puls, vorzugsweise im Bereich von 40 bis 60 mJ/mm² pro Puls liegt.

4. Kathetersystem nach einem der vorhergehenden Ansprüche, in welchem die optischen Fasern (4) des Faserbündels (3) in einer rohrförmigen Konfiguration angeordnet sind, um die Licht emittierende Oberfläche (6) als ringförmig aussehend zu gestalten.

5. Kathetersystem nach einem der vorhergehenden Ansprüche, in welchem die Zeitsteuereinrichtung (63) ferner eine Eingabeeinrichtung (63) zum Eingeben vorbestimmter Zeitwerte für die Emissionsintervalle (T1, T2) und die Unterbrechungsintervalle (B1) umfasst.

6. Kathetersystem nach einem der vorhergehenden Ansprüche, in welchem die Emissionsintervalle (T1, T2) jeweils wenigstens 2 s, vorzugsweise im Bereich von 2 bis 5 s liegen.

7. Kathetersystem nach einem der vorhergehenden Ansprüche, in welchem das Unterbrechungsintervall (B1) im Bereich von 1 bis 5 s liegt.

8. Kathetersystem nach einem der vorhergehenden Ansprüche, in welchem der pulsierende Lichtstrahl eine Pulsfrequenz von etwa 40 Hz hat.

9. Kathetersystem nach einem der vorhergehenden Ansprüche,
wobei der distale Teil des Katheters (1) mit einem Greifer (11) zum Greifen von Gewebe versehen ist;
wobei der Greifer (11) einen Hohlkanal (9) umfasst, der sich innerhalb des Faserbündels (3) der optischen Fasern (4) erstreckt und mit einer Vakuumquelle (10) verbunden werden kann, wobei das distale Ende des Kanals eine Saugöffnung (11) bildet; und
wobei die Saugöffnung (11) in einem Abstand proximal von der Licht emittierenden Oberfläche (6) angeordnet ist und eine Saugöffnung parallel zur Licht emittierenden Oberfläche (6) bildet.

10. Kathetersystem nach einem der vorhergehenden Ansprüche, in welchem der Laserkatheter (1) eine Stoppfläche (7) umfasst, welche sich um das Faserbündel (3) der optischen Fasern (4) herum erstreckt und in die distale Richtung gewandt ist, wobei die Stoppfläche (7) in einem Abstand proximal von der Licht emittierenden Oberfläche (6) angeordnet ist.

11. Kathetersystem nach Anspruch 10, ferner mit einem Ringelement (15), das Abmessungen hat, die einerseits für die Einführung des distalen Endes des Faserbündels (3) der optischen Fasern (4) durch das Ringelement (15) hindurch und andererseits für ein Verhindern des Durchgangs der Stoppfläche (7) durch das Ringelement (15) hindurch ausgebildet ist.

12. Kathetersystem nach Anspruch 11, mit einem Implantatgefäß (16), das Durchmesserabmessungen hat, welche einerseits einen Durchgang des Laserkatheters (1) und andererseits eine Einführung durch das Ringelement (15) hindurch erlaubt.

13. Kathetersystem nach Anspruch 12, in welchem das Implantatgefäß (16) ein künstliches Implantatgefäß ist.

14. Kathetersystem nach Anspruch 12 oder 13, in welchem ein Ende des Implantatgefäßes (16) durch das Ringelement (15) hindurch eingesetzt und über das Ringelement (15) zurückgeklappt ist und wobei der Winkel zwischen dem Implantatgefäß (16) und dem Teil (18) des zurückgeklappten Implantatgefäßes im Bereich von 90 bis 180 Grad liegt.

15. Kathetersystem nach einem der vorhergehenden Ansprüche, in welchem die Licht emittierende Oberfläche (6) im Wesentlichen senkrecht zur Längsrichtung des Katheters (1) verläuft.

## Revendications

1. Système cathéter, comprenant :
- un cathéter à laser (1) doté d'un faisceau de fibres (3) de fibres optiques (4) ayant des extrémités distales (5) définissant une surface émettrice de lumière (6) pour émettre un faisceau de lumière pulsée dans la direction distale du cathéter (1) ; et
- un appareil à laser (43), comprenant au moins un laser pour fournir de la lumière aux fibres optiques (4), dans lequel l'appareil à laser (43) est un laser à excimère fournissant une lumière ultraviolette dans la plage de 240 à 340 nm ;
dans lequel le faisceau de lumière pulsée a une fréquence de pulsation dans la plage de 25 à 80 Hz ;
dans lequel le faisceau de lumière pulsée a une largeur d'impulsion dans la plage de 50 à 300 ns à pleine largeur à mi-hauteur (F.W.H.M) ;
dans lequel le système est préréglé ou ajusté pour émettre un faisceau de lumière pulsée avec une puissance d'ablation d'au moins 40 mJ/mm² par pulsation au niveau de l'emplacement de la surface émettrice de lumière (6) ;
dans lequel le système cathéter (1) comprend en outre un dispositif de synchronisation (63) pour définir des intervalles d'émission (T1, T2) et des intervalles de pause (B1) à des valeurs temporelles prédéfinies ; et
dans lequel le système cathéter (1) est agencé pour émettre un faisceau de lumière pulsée durant lesdits au moins deux intervalles d'émission (T1, T2), dans lequel deux intervalles d'émission consécutifs (T1, T2) sont séparés par un intervalle de pause (B1) d'au moins 1 s, durant lequel l'émission du faisceau de lumière pulsée est interrompue.

2. Système cathéter selon la revendication 1, comprenant en outre :
- un dispositif d'ajustement (61) pour définir une puissance d'ablation du faisceau de lumière pulsée ;
- un capteur de puissance (65) pour mesurer la puissance d'ablation et pour générer un signal de puissance représentatif de la puissance d'ablation mesurée ; et
- un dispositif de commande pour commander le dispositif d'ajustement en réponse au signal de puissance pour définir ladite puissance d'ablation à une valeur d'au moins 40 mJ/mm² par impulsion au niveau de l'emplacement de la surface émettrice de lumière (6).

3. Système cathéter selon l'une quelconque des revendications précédentes,
dans lequel la puissance d'ablation est dans la plage de 40 à 100 mJ/mm² par impulsion, de préférence dans la plage de 40 à 60 mJ/mm² par impulsion.

4. Système cathéter selon l'une quelconque des revendications précédentes,
dans lequel les fibres optiques (4) du faisceau de fibres (3) sont agencées en une configuration tubulaire pour définir ladite surface émettrice de lumière (6) comme étant de forme annulaire.

5. Système cathéter selon l'une quelconque des revendications précédentes, dans lequel le dispositif de synchronisation (63) comprend en outre un dispositif d'entrée (63) pour entrer des valeurs temporelles prédéfinies pour les intervalles d'émission (T1, T2) et les intervalles de pause (B1).

6. Système cathéter selon l'une quelconque des revendications précédentes, dans lequel les intervalles d'émission (T1, T2) sont d'au moins 2 s, de préférence dans la plage de 2 à 5 s.

7. Système cathéter selon l'une quelconque des revendications précédentes, dans lequel l'intervalle de pause (B1) est dans la plage de 1 à 5 s.

8. Système cathéter selon l'une quelconque des revendications précédentes, dans lequel le faisceau de lumière pulsée a une fréquence de pulsation d'environ 40 Hz.

9. Système cathéter selon l'une quelconque des revendications précédentes,
dans lequel la partie distale du cathéter (1) est dotée d'un dispositif de préhension (11) pour saisir le tissu ;
dans lequel le dispositif de préhension (11) comprend un canal creux (9) s'étendant à l'intérieur du faisceau de fibres (3) de fibres optiques (4) et pouvant être raccordé à une source de vide (10), l'extrémité distale du canal définissant une bouche d'aspiration (11) ; et
dans lequel la bouche d'aspiration (11) est agencée à une distance proximalement de la surface émettrice de lumière (6) et définit une surface d'aspiration parallèle à la surface émettrice de lumière (6).

10. Système cathéter selon l'une quelconque des revendications précédentes, dans lequel le cathéter à laser (1) comprend une surface d'arrêt (7) s'étendant autour du faisceau de fibres (3) de fibres optiques (4) et orientée dans la direction distale, la surface d'arrêt (7) étant agencée à une distance proximalement de la surface d'émission de lumière (6).

11. Système cathéter selon la revendication 10, comprenant en outre
un élément annulaire (15) ayant des dimensions adaptées, d'une part, pour insérer l'extrémité distale du faisceau de fibres (3) de fibres optiques (4) dans ledit élément annulaire (15) et, d'autre part, empêcher le passage de la surface d'arrêt (7) à travers ledit élément annulaire (15).

12. Système cathéter selon la revendication 11, comprenant en outre
une greffe vasculaire (16) ayant des dimensions de diamètre permettant, d'une part, le passage du cathéter à laser (1) et, d'autre part, l'insertion à travers ledit élément annulaire (15).

13. Système cathéter selon la revendication 12, dans lequel la greffe vasculaire (16) est une greffe vasculaire artificielle.

14. Système cathéter selon la revendication 12 ou la revendication 13,
dans lequel une extrémité de la greffe vasculaire (16) est insérée dans ledit élément annulaire (15) et repliée sur ledit élément annulaire (15), et dans lequel l'angle entre la greffe vasculaire (16) et la partie (18) de la greffe vasculaire repliée est dans la plage de 90 à 180 degrés.

15. Système cathéter selon l'une quelconque des revendications précédentes, dans lequel la surface émettrice de lumière (6) est sensiblement perpendiculaire à la direction longitudinale du cathéter (1).
